# EUROPEAN PATENT SPECIFICATION

(11) **EP 2 803 682 B1**
(45) Date of publication and mention of the grant of the patent: **23.03.2016**
(21) Application number: 13179542.9
(22) Date of filing: 07.08.2013
(51) Int. Cl.: G01T 1/04, C08F 220/58

(54) **Polymerizable composition, method of making the composition and its use in a dosimeter**
Polymerisierbare Zusammensetzung, Verfahren zur Herstellung der Zusammensetzung und ihre Verwendung in einem Dosimeter
Composition polymérisable, procédé de fabrication de la composition et son utilisation dans un dosimètre

(30) Priority: 13.05.2013 US 201313892484
(43) Date of publication of application: 19.11.2014
(73) Proprietor: King Abdulaziz City for Science and Technology, Riyadh 11442 (SA)
(72) Inventor: Basfar, Ahmed Ali, 11442 Riyadh (SA); Moftah, Belal, 11211 Riyadh (SA); Rabaeh, Khalid A., 21166 Irbid (JO); Almousa, Akram A., 11211 Riyadh (SA)
(74) Representative: Gulde & Partner

(56) References cited:
- EP-A1- 2 351 779
- US-A- 5 321 357

## Description

### FIELD OF INVENTION

The present invention describes a novel composition of a polymerizable gel, a method of making the polymerizable composition and its use for dosimetric analysis. More specifically it relates to radiation-induced polymerization of normal and normoxic 3D polymer gel dosimeters for planning of radiation treatment.

### BACKGROUND

Advanced treatment techniques such as intensity modulated radiotherapy (IMRT), brachytherapy and stereotactic radio-surgery (SRS) and boron nuclear capture therapy (BNCT) require highly conformal 3D dose distributions. Polymer gel dosimeters have shown to be a very useful tool in the verification of radiotherapy treatment planning system (TPS), as it offers a number of advantages over traditional single point-dose dosimeters such as ionization chambers and thermo-luminescent dosimeters (TLD) and two-dimensional (2D) radiographic films. The advantages include independence of radiation direction, radiological soft tissue equivalence, integration of dose for a number of sequential treatment fields, and perhaps most significantly, evaluation of a complete tumor volume at once (De Deene, Y.; 2009). MRI was suggested as the first method to measure 3D complex dose distributions produced by ionizing radiation absorbed in tissue-equivalent (TE) gelatin by Gore, J. C. et. al., 1984.

The first proposed TE gel was the chemical solution of ferrous sulfate (Fricke) dosimeter. Upon irradiation, radiation-induced free radicals produced by the radiolysis of water oxidize ferrous ions (Fe2+) to ferric ions (Fe3+) that alters the magnetic moment of the metal ion. As a result, the spin-spin relaxation times of water protons in the aqueous gel are reduced and consequently the NMR spin-spin relaxation rate (R2) of the protons increases (De Deene, Y. et. al.; 2006). A major limitation in Fricke gel systems is the diffusion of ions Fe3+ within the dosimeter over time, resulting in the change of dose distribution with time, i.e. MRI scans must be taken immediately after irradiation.

However, none of the above-discussed references discloses or suggests a relatively inexpensive but highly effective 3D polymer composition for dosimetric use. Accordingly, there exists a need in the art to overcome the deficiencies and limitations described hereinabove.

### SUMMARY OF THE INVENTION

The present invention relates to polymer gel dosimeters containing N-(Hydroxymethyl) acrylamide (NHMA). These polymer gels are tissue equivalent polymer gels whose nuclear magnetic resonance (NMR) and optical properties change with radiation dose. They may provide unique advantages for measuring radiation dose distributions in three-dimensions (3D).

Similar copolymers are disclosed in EP 2351779.

In one embodiment, a polymer gel dosimeters was made by polymerizing N-(Hydroxymethyl)acrylamide (NHMA) in a glove-box under nitrogen atmosphere (PNHMAG) and under a fume hood in normal atmospheric conditions (NHMAGAT). In another embodiment, the polymerized gels were irradiated with (Varian medical systems, USA) at absorbed doses up to 20 Gy for diagnosis. In another embodiment, the characterization of the polymer gel was performed using the nuclear magnetic resonance (NMR) spin-spin relaxation rate (R2) for water proton surrounding polymer formation and the degree of polymerization of both gels was done. The change in R2 corresponding to the amount of polymer formation in both gels increases gradually with absorbed dose up to 20 Gy. It was found that there is no appreciable effect of dose rate and radiation energy on both polymer gel dosimeters. Spectrophotometer was used to investigate degree of whiteness of irradiated samples of NHMAGAT which is associated with the degree of polymerization of polymer gel dosimeters. The variation in absorbance corresponding to the amount of polymer formation in NHMAGAT gels increases gradually with absorbed dose up to 30 Gy. The optically dosimetric method also showed that there is no appreciable effect of dose rate and radiation energy on polymer gel dosimeters.

In one embodiment, the composition of the polymerizable gel comprises 2-5 % by weight Gelatin, 1-12 % by weight N-(Hydroxymethyl)acrylamide (NHMA), 1-4 % by weight N, N-methylene-bis-acrylamide (BIS), 0-0.1 mM Hydroquinone (HQ), 1-50 mM Tetrakis (hydroxymethyl)phosphonium chloride (THPC),and Ultra-pure de-ionized water as a solvent.

Preferably, the composition further comprises 1-50 mM Tetrakis (hydroxymethyl)phosphonium chloride (THPC).

According to a preferred embodiment the composition comprises 3 % by weight gelatin, 0.02 mM Hydroquinone, and 3 % by weight N,N-methylene-bis-acrylamide (BIS).

Preferably, the composition comprises 4-8 % by weight N-(Hydroxymethyl)acrylamide (NHMA).

The amount of of Tetrakis (hydroxymethyl)phosphonium chloride (THPC) is preferably about 5 mM.

In one embodiment, the polymerizable composition is made by several steps including soaking between 2-5 % by weight of gelatin for 10 minutes in deionized water to make a mixture of the gelatin and deionized water; heating the mixture of the gelatin and the deionized water for 1 hour at 50°C to make a gelatin solution; cooling the gelatin solution to 40°C; adding between 1-12 % by weight of N-(Hydroxymethyl)acrylamide (NHMA) and between 0-0.1 mM Hydroquinone (HQ) to the gelatin solution and mixing to make a second solution; cooling the second solution to 35°C; and adding between 1-50 mM of Tetrakis (hydroxymethyl)phosphonium chloride (THPC) to the second solution and forming a N-(Hydroxymethyl)acrylamide (NHMA) polymerizable gel and storing the polymerizable gel at 10°C in a refrigerator until further use for measuring irradiation results. In another embodiment, a chamber, in which the steps of making the gelatin solution, the second solution and of adding the THPC to form a normoxic N-(Hydroxymethyl)acrylamide (NHMA) polymer gel are conducted, is purged with nitrogen to remove oxygen during said steps.

Preferably, 3 % by weight of gelatin, 0.02 mM of Hydroquinone, and 3 % by weight of N,N-methylene-bis-acrylamide (BIS) are used.

For example, 4-8 % by weight of N-(Hydroxymethyl)acrylamide (NHMA) is used.

Preferably, 5 mM of Tetrakis (hydroxymethyl)phosphonium chloride (THPC) is added.

The polymerizable composition according to the invention is preferably used for measuring radiation doses, in particular for three-dimensionally measuring radiation dose distributions.

Another aspect of the present invention is directed to a 3D dosimeter comprising the polymerizable composition according to the invention.

In one embodiment, a method of testing the polymer gel is described. The composition and methods disclosed herein may be implemented in any means for achieving various aspects, and may be executed manually or automated using a computer. Other features will be apparent from the accompanying figures and from the detailed description that follows.

### BRIEF DESCRIPTION OF DRAWINGS

Example embodiments are illustrated by way of example in the accompanying figures, in which like references indicate similar elements:
**Fig. 1** shows the Relaxation rate (R₂) ofPNHMAG polymer gel as a function of the absorbed dose. R₂ of the gel increases gradually with increase of dose.
**Fig. 2** shows the Relaxation rate (R₂) ofPNHMAG polymer gels for different dose rates (200, 400 and 600 cGy/min) under 10 MV radiation energy as a function of absorbed dose.
**Fig. 3** shows the Relaxation rate (R₂) of PNHMAGA polymer gel for different radiation energies (6, 10 and 18 MV) at 600 Gy/min as a function of absorbed dose.
**Fig. 4** shows the Dose-R₂ ofPNHMAG dosimeters irradiated to 10 and 20 Gy as a function of storage time.
**Fig. 5** shows the Relaxation rate (R₂) of NHMAGAT (1, 2, 3) polymer gel at different anti-oxidant concentrations as a function of absorbed dose.
**Fig. 6a** **and** **6b** shows Dose response curves of NHMAGAT (4, 5, 6) polymer gel at different NHMA concentrations in the dose range (a) 0 to 10 Gy and (b) 0 to 20 Gy, respectively.
**Fig. 7** shows Dose sensitivity values for different NHMA concentrations at 3 % gelatin as function of absorbed dose. The sensitivity value increases gradually with increasing NHMA concentrations from 4 to 8 wt%, leading to the increase of polymerization of NHMAGAT gel dosimeters.
**Fig. 8** shows the Relaxation time (R₂) of NHMAGAT-6 polymer gel for 6 and 12 Gy as a function of scanning temperature.
**Fig. 9** shows the Relaxation time (R₂) of NHMAGAT-6 polymer gel for different dose rates (2, 4 and 6 Gy/min) under 10 MV of radiation energy as function of the absorbed dose.
**Fig. 10** shows the Relaxation time (R₂) of NHMAGAT-6 polymer gel for different radiation energies (6, 10 and 18 MV) at 6 Gy/min as function of absorbed dose.
**Fig. 11** shows the Relaxation (R₂) of NHMAGAT dosimeters irradiated to 0, 10 and 20 Gy as a function of storage time.
**Fig. 12** shows irradiated samples of NHMAGAT polymer gel dosimeter at increasing doses.
**Fig. 13** shows the Absorbance at 500 nm of NHMAGAT (4, 5 and 6) polymer gel at different NHMA concentrations as function of absorbed dose.
**Fig. 14** shows the Absorbance at 500 nm of NHMAGAT-5 polymer gel for 6 and 12 Gy as a function of irradiation temperature.
**Fig. 15** shows the Absorbance at 500 nm of NHMAGAT-6 polymer gel for different dose rates (2, 4 and 6 Gy/min) under 10 MV of radiation energy.
**Fig. 16** shows the Absorbance at 500 nm of NHMAGAT-6 polymer gel for different radiation energies (6, 10 and 18 MV) at 6 Gy/min as function of absorbed dose.
**Fig. 17** shows the Absorbance at 500 nm NHMAGAT-6 dosimeters irradiated to 0, 10 and 20 Gy as a function of storage time.

Other features of the present embodiments will be apparent from the accompanying figures and from the detailed description that follows.

### DETAILS OF THE INVENTION

Several embodiments for the 3D gel composition and method of use are disclosed. Although the present invention is described with reference to specific example embodiments, it will be evident that various modifications and changes may be made to these embodiments without departing from the present invention as defined by the claims.

This limitation led to the investigation of alternative gel dosimetry systems which could be used in conjunction with MRI scans and based on a set of several simple considerations, i.e. the gels must be tissue equivalent and mechanically strong, the monomers must be highly reactive so that a significant amount of the polymer should be formed per unit concentration of free radical initiators produced by water radiolysis. A relatively high cross-linking density of the polymer is clearly needed to produce significant NMR relaxation effect of water protons (Maryanski, et.al. 1997). The 3D gel dosimeters are used to plan the radiotherapy treatment of patients to evaluate the distribution of absorbed dose in a phantom which simulates the human organ to be treated. So, the use of these gels is in the planning phase of the treatment before exposing the patient to radiotherapy. Several other uses are envisioned such as developing NHMAGAT gel dosimeters for 3D radiotherapy treatment planning using MRI scan and Developing NHMAGAT gel dosimeters for 3D nuclear medicine using gamma camera.

Radiation-induced changes in a polymer gel dosimeter manufactured using 2-hydroxyethylacrylate (HEA) and BIS were investigated using magnetic resonance imaging (MRI) (Gustavsson, H., et.al. 2004). The gels were manufactured in a glove box facility, which was purged with nitrogen at least 18 hours before and continuously during the mixing of the gel. It was observed that the dose response decreases with increasing the concentration of gelatin while it increases with increasing the concentration of co-monomer. Gelatin molecules consume some of the free radicals created in water radiolysis. Thus, a lowered gelatin concentration will lead to an increased number of radicals available for initiation of the polymerization process.

The first normoxic gel, known as MAGIC (methacrylic and ascorbic acid in gelatin initiated by copper (II) sulphate, ascorbic acid and hydroquinone) was made, which can be produced, stored and irradiated in a normal or normoxic environment without purging nitrogen (Fong, P. M. et.al.,2001).

Venning A. J., et.al. (2005) investigated PAGAT polymer gel dosimeter using magnetic resonance imaging. Polyacrylamide gel (PAG) dosimeter consists of co-monomers (AA and BIS) with the anti-oxidant Tetrakis(hydroxymethyl)phosphonium chloride (THPC) and the polymerization inhibitor hydroquinone (HQ) to form the normoxic PAGAT polymer gel dosimeter. PAGAT polymer gel displayed a high degree of spatial stability over the 24 days period, good dose resolution and spatial integrity.

Mariani, M. et. al. (2007) introduced a new measurement technique for polymer gel dosimeter using spectroscopy meter. PAGAT polymer gel dosimeters were used in this study based on the work of Venning A J, et.al.(2005). Dosimeter-gel samples were optically analyzed by measuring the visible-light transmittance through gel samples by an UV-VIS spectrophotometer. It was found that the dose response increased linearly with increase of absorbed dose up to 20 Gy. Recent developments of complex radiotherapy treatment techniques have emphasized the need for a dosimetric system with the ability to measure absorbed dose distributions in three dimensions and with high spatial resolution. Therefore, normal and normoxic polymer gel dosimeters containing N-(Hydroxymethyl)acrylamide are introduced in this invention for radiation therapy with low toxicity, low cost and high dose response compared to the commercial product based on PAGAT (Maryanski, M., et.al., 1994).

The normal N-(Hydroxymethyl)acrylamide polymer gels (PNHMAG) were synthesized in a nitrogen atmosphere glove-box according to Gustavsson, H. (2004). The PNAHMAG dosimeters were composed of 8 % N-(Hydroxymethyl)acrylamide (NHMA) monomer, 3 % N,N-methylene-bis-acrylamide (BIS) cross-linker, 4 % gelatin (Type A, bloom 300) and 0.02 mM hydroquinone (HQ). All co-chemicals were obtained from SIGMA Chemical Co. (St. Louis, Mo, USA). The chamber was purged with nitrogen at least 5 hours continuously during the mixing of the gel in order to expel oxygen that inhibits polymerization prior to gamma irradiation. After deoxygenating the water for a minimum of 2 hours, the gelatin was added to the ultra-pure de-ionized water and left to soak for 10 minutes before heating to 50 °C for 1 hour. While continuously stirring, BIS was added and stirred until completely dissolved. After the solution was cooled down to 40 °C, NHMA and HQ were added respectively and stirred until completely dissolved. For characterization study, the final polymer gels were filled into 10 mm NMR tube (Wilmad glass, Buena, NJ, USA) and sealed. All gels were stored in a refrigerator (10 °C) overnight prior to irradiation.

The irradiations were performed using a 6 MV photon beam of a medical linear accelerator (Varian medical systems, USA) with dose rate of 600 cGy/min calibrated using ionization chamber. Each sample filled with gel was placed in a 30 × 30 × 30 cm3 cubic water phantom. The sample was irradiated with beam field size 10 × 10 cm2 with different doses at 5 cm depth and 100 cm (SSD). The sample was transferred back to the refrigerator and kept for about 24 hours before NMR measurements of relaxation Rate (R2). The relaxation Rate (R2) measurements were performed using 0.5 Tesla NMR (Bruker, Germany). The irradiated polymer gel sample was put in NMR tube (1 cm diameter and 20 cm height) and lowered into magnetic box (probe head). A standard multi-Spin-Echo Carr Purcell Meiboom Gill (CPMG) sequence was used to measure R2. UV/VIS spectrophotometer (model Lambda 850, from Perkin-Elmer, USA) was also used to measure the absorption spectra of irradiated gel samples in the wavelength range from 350 - 650 nm. Three samples at each absorbed dose were measured, but no significant differences in their characteristics were found during measurements. The temperature during measurements was 22 ± 0.5°C.

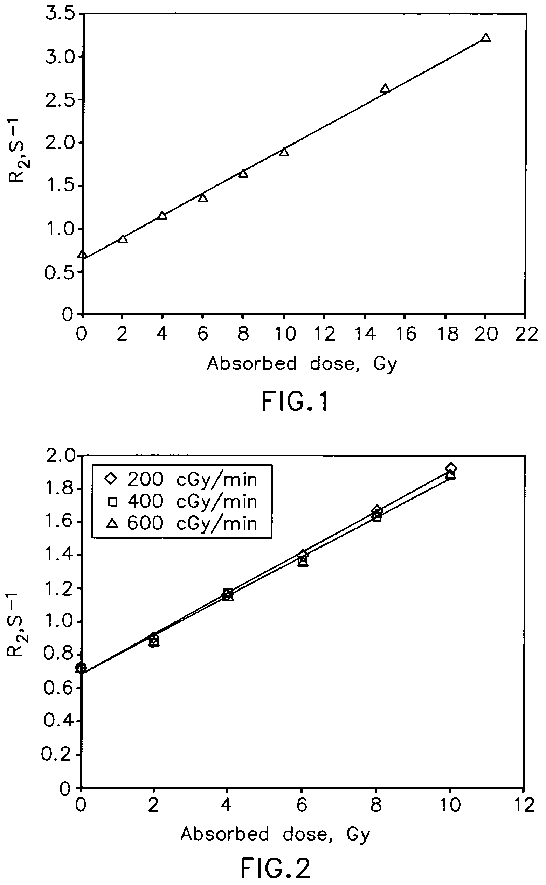

The dose response of the gel dosimeter was investigated by preparing PNHMAG dosimeters containing 4 % gelatin, 3 % Bis, 8 % NHMA, and 0.02mM HQ. The change in the proton relaxation rate R2 versus absorbed dose for polymerization of PNHMAG is shown in Fig. 1. The dose response of the gel increases gradually with increase of dose. As the dose increases, more radiation-induced free monomer radicals were generated due to the breakage of C=C bonds of the co-monomers, resulting in an increase of polymer gel formed. Linearity of dose response can be seen over the region up to 20 Gy.

The effect of dose rate on the response of PNHMAG polymer gel dosimeters was investigated using 200, 400 and 600 cGy/minute under 10 MV radiation energy. The samples were irradiated for absorbed doses of 2, 4, 6, 8 and 10 Gy. Three dosimeters were irradiated for each dose point. It was found that there is no appreciable effect of dose rate on PNHMAG polymer gel dosimeters (see Fig. 2).

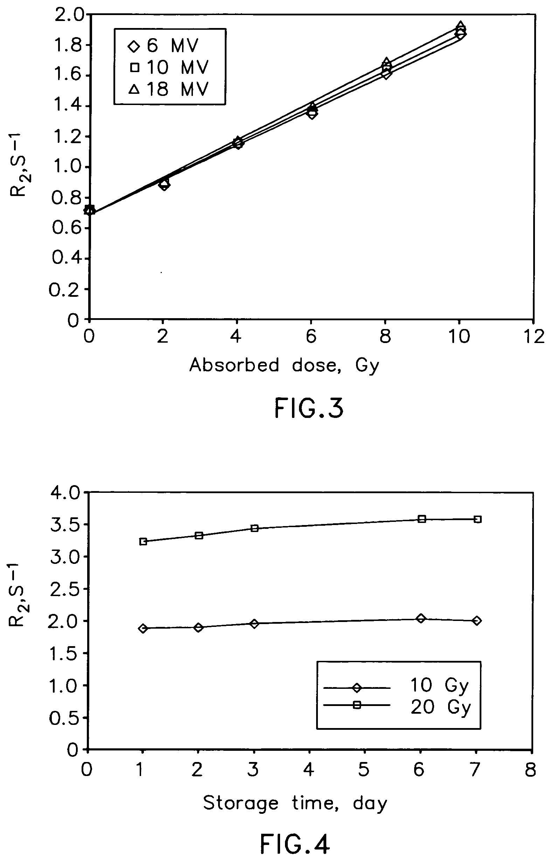

The effect of energy on the response of PNHMAG polymer gel dosimeters was investigated using 6, 10 and 18 MV at 600 Gy/min. The samples were irradiated for absorbed doses of 2, 4, 6, 8 and 10 Gy. Three dosimeters were irradiated for each dose point. It was found that there is no appreciable effect of radiation dose on PNHMAG polymer gel dosimeters (see Fig. 3).

The stability of gel dosimeters was tested by irradiating PNHMAG dosimeters to 10 and 20 Gy and storing in a refrigerator (10 °C). A set of three samples was used for each dose. The results show no change (less than ±4.7 %; 1σ) in the absorbance of the gel dosimeters up to 7 days (see Fig. 4).

Due to the complexity of handling normal gel dosimeter (PNHMAG) in a glove-box under nitrogen atmosphere, normoxic polymer gel dosimeters containing different concentrations of NHMA are introduced in this invention. The N-(Hydroxymethyl)acrylamide polymer gels were synthesized under a fume hood in normal atmospheric conditions. The NAHMAGAT dosimeters were composed of N-(Hydroxymethyl)acrylamide (NHMA) monomer, N,N-methylene-bisacrylamide (BIS) cross-linker, gelatin (Type A, bloom 300), hydroquinone (HQ) and tetrakis(hydroxymethyl)phosphonium chloride (THPC). All co-chemicals were obtained from SIGMA Chemicals Co. (St. Louis, Mo, USA). The gelatin was added to the ultra-pure de-ionized water and left to soak for 10 minutes before heating to 50 °C for 1 hour. After the solution was cooled down to 40 °C, NHMA and HQ were added, respectively and stirred until completely dissolved. After the solution was cooled down to 35 °C, THPC was added as antioxidant. For NMR and optical characterization studies, the final polymer gels were filled into 10 mm NMR tube and 3 mm cuvette, respectively and sealed. All gels were stored in a refrigerator (10 °C) overnight prior to irradiation. The irradiation of NHMAGAT and NMR measurements of the gels were performed as mentioned previously for PNHMAG. UV/VIS spectrophotometer was used to measure the absorbance of spectra in the near infrared (700-1100 nm), visible (350-700 nm) and ultra violet (190-350) nm regions. The absorption spectra of irradiated gel samples in the wavelength range from 350 - 650 nm were measured using UV/VIS spectrophotometer, model Lambda 850, from Perkin-Elmer, USA. Three samples at each absorbed dose were measured, but no significant differences in their characteristics were found during measurements.

It was observed that after adding THPC to the NHMA gel dosimeter, the gels set rapidly at room temperature. The jellification time decreases with increase of THPC concentration as listed in **Table 1.**

**Table 1: Jellification time of NHMAGAT (7 % NHMA, 3 % BIS, 5 % Gelatin and various concentrations of THPC).**

| THPC (mM) | Jellification time (minute) |
|---|---|
| 5 | 8-9 |
| 10 | 7-8 |
| 15 | 6-7 |
| 20 | 5-6 |
| 30 | 4-5 |

Due to very small jellification times of the investigated NHMAGAT dosimeters which leads to limited time to fill dosimetry vials and less uniformity of the gcl dosimeter, low gelatin concentrations of NHMAGAT were studied as shown in Table 2.

**Table 2: Jellification time of NHMAGAT (7 % NHMA, 3 % BIS, 5-20 mM THPC and various gelatin concentrations).**

| Gelatin (%) | Jellification time (minute) |
|---|---|
| 5 | 5-9 |
| 4 | 12-15 |
| 3 | 90-95 |
| 2.5 | 420-450 |

The effect of anti-oxidant (THPC) concentration on the response of the NHMAGAT dosimeter was investigated by preparing different compositions of gel dosimeters as listed in Table 3.

**Table 3: Compositions of NHMAGAT polymer gels based on 3 % gelatin and 5-20 mM THPC.**

| Formulation code | Water (wt%) | Gelatin (wt%) | NHMA (wt%) | BIS (wt%) | THPC (mM) |
|---|---|---|---|---|---|
| NHMAGAT-1 | 86 | 3 | 8 | 3 | 5 |
| NHMAGAT-2 | 86 | 3 | 8 | 3 | 10 |
| NHMAGAT-3 | 86 | 3 | 8 | 3 | 20 |

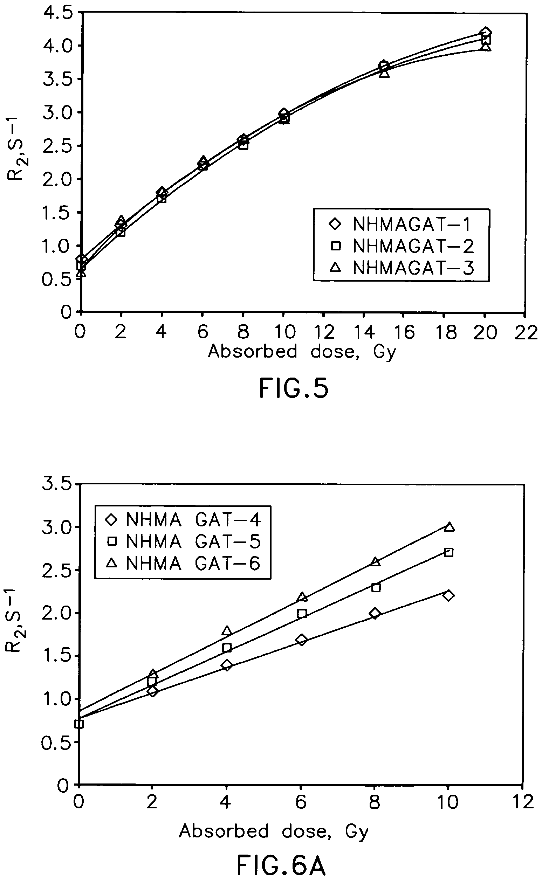

The dose response curves were established in terms of change in the proton relaxation rate *R*₂ versus the absorbed dose. Fig. 5 shows the dose response of different concentrations of THPC in the dose range 0-20 Gy. The results show that the dose response is not affected by the increase of THPC concentration. THPC concentration of 5 mM with 3 wt% gelatin were used in all dosimeters to ensure sufficient THPC for oxygen scavenging, while maintaining adequate gel stiffness.

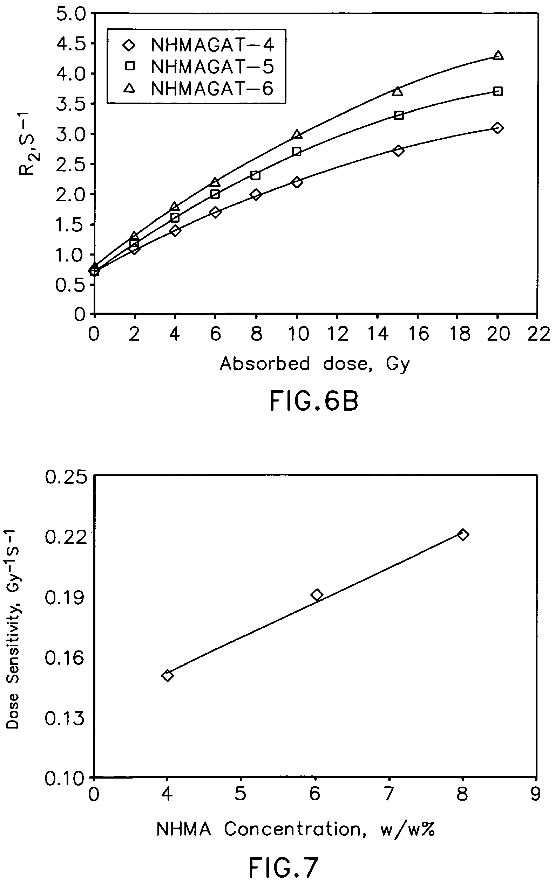

The effect of the NHMA concentration on the response of the gel dosimeter was investigated by preparing different compositions of NHMA GAT dosimeters as listed in Table 4. The change in the proton relaxation rate *R*₂ versus absorbed dose for polymerization of NHMAGAT for NHMA concentration from 4 to 8 wt% is shown in Fig. 6 (A) and (B). The dose response of the gel increases gradually with increase of dose, which can be seen from an increase of the individual relative absorbance-dose curves (see Fig. 6 B). As the dose increases, more radiation-induced free monomer radicals are generated due to the breakage of C=C bonds of the co-monomers, resulting in an increase of polymer gel formed. The results show that the relaxation rate *R₂* increases significantly with increase of NHMA concentration. Linearity of dose response can be seen over the region up to 10 Gy (Fig. 6 (A)). Beyond 10 Gy the response changes towards saturation due to the consumption of co-monomers.

**Table 4: Compositions of NHMAGAT polymer gels based on 3wt% gelatin and 4-8 wt% NHMA.**

| Formulation code | Water (wt%) | Gelatin (wt%) | NHMA (wt%) | BIS (wt%) | HQ (mM) | THPC (mM) |
|---|---|---|---|---|---|---|
| NHMAGAT-4 | 91 | 3 | 4 | 3 | 0.02 | 5 |
| NHMAGAT-5 | 89 | 3 | 6 | 3 | 0.02 | 5 |
| NHMAGAT-6 | 86 | 3 | 8 | 3 | 0.02 | 5 |

The dose sensitivity was taken from the slope of linear plot of dose D versus R₂ of Fig. 6(A). The results show that the sensitivity value increases gradually with increasing NHMA concentrations from 4 to 8 wt%, leading to the increase of polymerization ofNHMAGAT gel dosimeters as shown in Fig. 7.

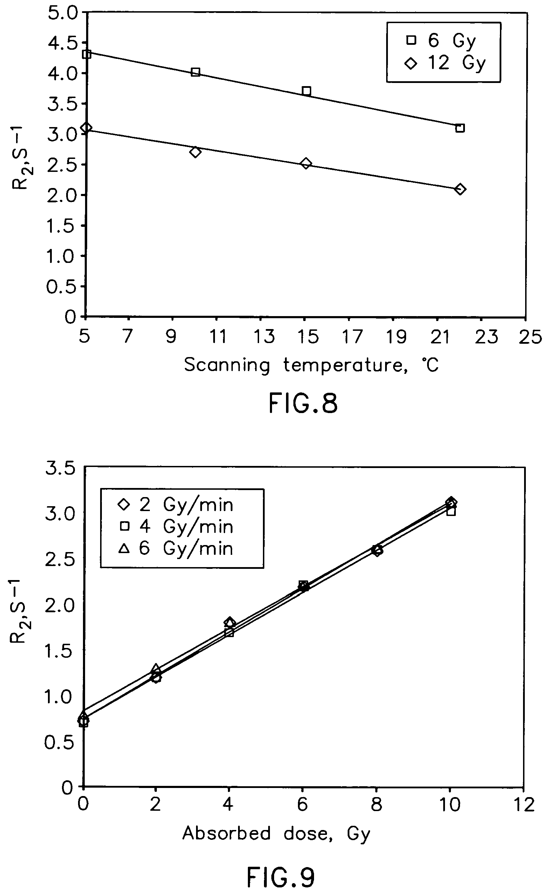

The effect of scanning temperature of NMR on relaxation rate *R*₂ of NHMAGAT polymer gel dosimeters was investigated by irradiating samples of formulation code NHMAGAT-6 (see Table 4) to 6 Gy and 12 Gy. A set of three samples were used for each temperature. The results show that *R*₂ increases upon cooling the gel during the NMR measurement (Fig. 8) due to slow down of the segmental motions of the polymer chains which lead to an increase of the correlation times of the semi-solid protons. The response of the dosimeters has to be corrected under actual processing conditions (ASTM. Standard guide for performance characterization of dosimeters and dosimetry systems for use in radiation processing. ASTM E2701-09).

The effect of the dose rate on the response of NHMAGAT polymer gel dosimeters was investigated using 2, 4 and 6 Gy/minute under 10 MV radiation energy. NHMAGAT-6 samples (see Table 4) were irradiated for absorbed doses of 2, 4, 6, 8 and 10 Gy. Three dosimeters were irradiated for each dose point. It was found that there is no appreciable effect of dose rate on NHMAGAT polymer gel dosimeters (see Fig. 9).

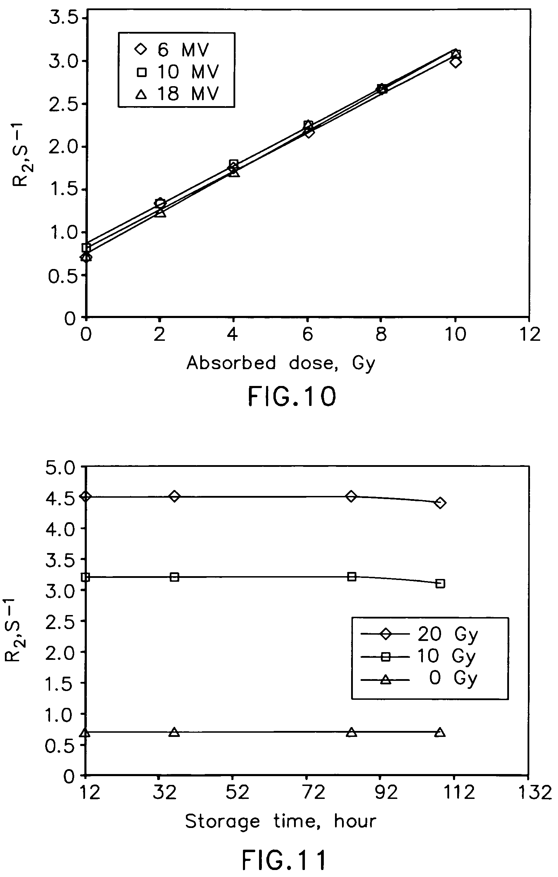

The effect of energy on the response ofNHMAGAT polymer gel dosimeters was investigated using 6, 10 and 18 MV at dose rate of 6 Gy/minute. NHMAGAT-6 samples (see Table 4) were irradiated for absorbed doses of 2, 4, 6, 8 and 10 Gy. Three dosimeters were irradiated for each dose point. It was found that there is no appreciable effect of radiation dose on NHMAGAT polymer gel dosimeters (see Fig. 10).

The stability of gel dosimeters was tested by irradiating NHMAGAT-6 (see Table 4) to 0, 10 and 20 Gy and storing in a refrigerator (10 °C). A set of three samples was used for each dose. The results show no change (less than ±2%; 1σ) in the absorbance of the gel dosimeters up to 108 hours (see Fig. 11).

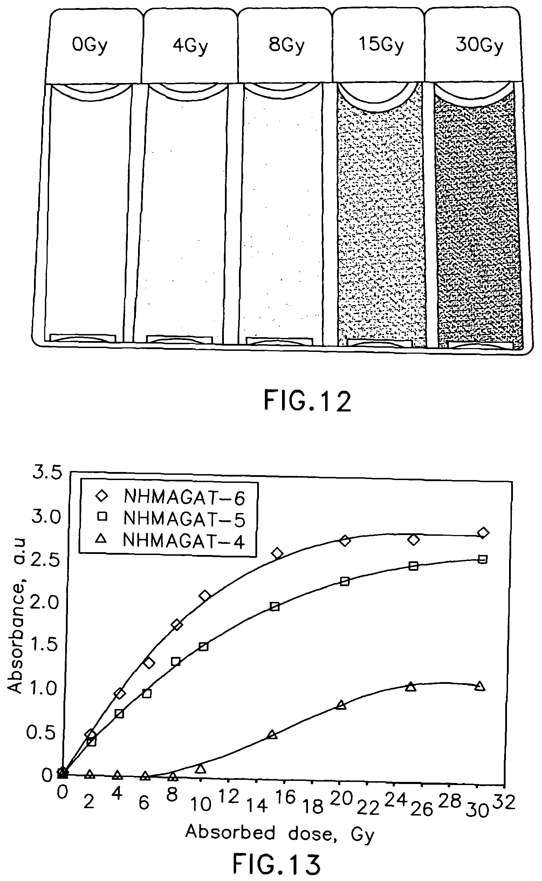

For further characterization of NHMAGAT polymer gels, spectrophotometer method was used to confirm NMR measurements. Upon irradiation, the color of the polymer gels changed to whitish along radiation beam as shown in Fig. 12, indicating that polymerization process has taken place in the dosimeters due to crosslinking between the co-monomers. The degree of whiteness intensity is dependent on the increase of dose and could be analyzed using the spectrophotometer. Therefore, the effect of the NHMA concentrations on the response of the gel dosimeter was investigated by preparing different compositions of NHMAGAT dosimeters as listed in Table 4. The dose response curves were established in terms of change in absorption measured at 500 nm versus the absorbed dose. The variation of absorbance versus absorbed dose for polymerization of NHMAGAT for NHMA concentration from 4 to 8 wt% is shown in Fig. 13. The dose response of the gel increases gradually with increase of dose, which can be seen from an increase of the individual relative absorbance-dose curves (see Fig. 13). As the dose increases, more radiation-induced free radicals were generated due to the breakage of C=C bonds of the co-monomers, resulting in an increase of polymer gel formed. The results show that the relaxation rate *R₂* increases significantly with increase of NHMA concentration.

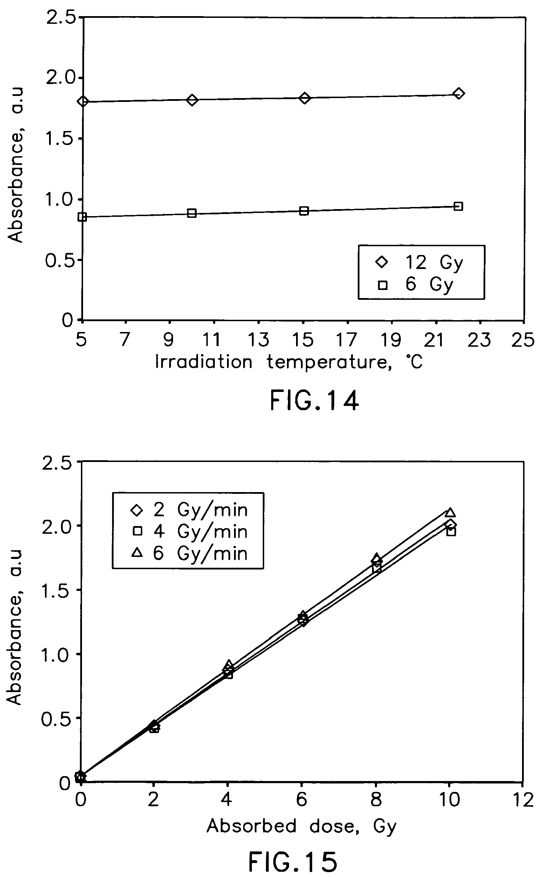

The effect of irradiation temperature ofNHMAGAT polymer gel dosimeters was investigated by irradiating samples of formulation code NHMAGAT-5 (see Table 4) to 6 Gy and 12 Gy at different temperatures during irradiation. A set of three samples was used for each temperature. The results show that dosimeters depend slightly on irradiation temperature (see Fig. 14). The response of the dosimeters has to be corrected under actual processing conditions (ASTM. Standard guide for performance characterization of dosimeters and dosimetry systems for use in radiation processing. ASTM E2701-09).

The effect of dose rate on the response of NHMAGAT polymer gel dosimeters was investigated using 2, 4 and 6 Gy/minute under 10 MV of radiation energy. NHMAGAT-6 samples (see Table 4) were irradiated for absorbed doses of 2, 4, 6, 8 and 10 Gy. Three dosimeters were irradiated for each dose point. It was found that there is no appreciable effect of dose rate on NHMAGAT polymer gel dosimeters (see Fig. 15).

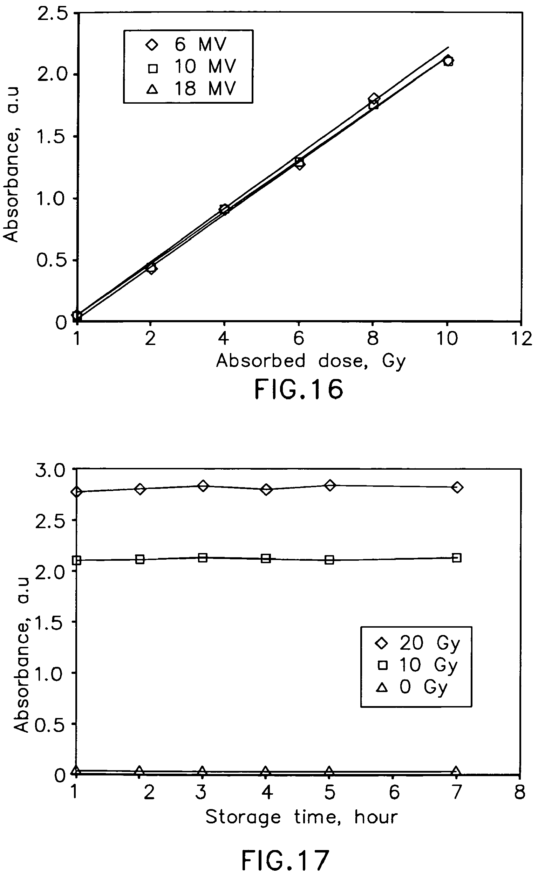

The effect of energy on the response ofNHMAGAT polymer gel dosimeters was investigated using 6, 10 and 18 MV at 6 Gy/min dose rate. NHMAGAT-6 samples (see Table 4) were irradiated for absorbed doses of 2, 4, 6, 8 and 10 Gy. Three dosimeters were irradiated for each dose point. It was found that there is no appreciable effect of radiation dose on NHMAGAT polymer gel dosimeters (see Fig. 16).

The stability of gel dosimeters was tested by irradiating NHMAGAT-6 to 0, 10 and 20 Gy and storing in a refrigerator (10 °C). A set of three samples was used for each dose. The results show no change (less than ±2%; 1σ) in the absorbance of the gel dosimeters up to 7 days (see Fig. 17).

In addition, it will be appreciated that the novel polymer for dosimetric use disclosed herein may be embodied using means for achieving better quality images for medical use and diagnosis. Accordingly, the specification and drawings are to be regarded in an illustrative rather than a restrictive sense.

## Claims

1. A polymerizable composition for use in a 3D gel dosimeter, comprising:
2-5 % by weight of Gelatin;
1-12 % by weight of N-(Hydroxymethyl)acrylamide (NHMA);
1-4 % by weight of N,N-methylene-bis-acrylamide (BIS);
0-0.1 mM of Hydroquinone (HQ) and
Ultra-pure de-ionized water as a solvent.

2. The composition of claim 1, further comprising:
1-50 mM Tetrakis (hydroxymethyl)phosphonium chloride (THPC).

3. The composition of claim 1 or 2, comprising 3 % by weight of gelatin, 0.02 mM Hydroquinone, and 3 % by weight ofN,N-methylene-bis-acrylamide (BIS).

4. The composition of any one of the preceding claims, comprising 4-8 % by weight ofN-(Hydroxymethyl)acrylamide (NHMA).

5. The composition of claim 2, comprising 5 mM of Tetrakis (hydroxymethyl)phosphonium chloride (THPC).

6. A method of making a polymerizable composition, comprising:
soaking 2-5 % by weight of gelatin for 10 minutes in deionized water to make a mixture of the gelatin and deionized water;
heating the mixture of the gelatin and the deionized water for 1 hour at 50 °C to make a gelatin solution;
cooling the gelatin solution to 40 °C;
adding 1-12 % by weight of N-(Hydroxymethyl)acrylamide (NHMA) and 0-0.1 mM of Hydroquinone (HQ) to the gelatin solution and mixing to make a second solution;
cooling the second solution to 35°C; and
adding 1-50 mM of Tetrakis (hydroxymethyl)phosphonium chloride (THPC) to the second solution and forming an N-(Hydroxymethyl)acrylamide (NHMA) polymerizable gel and storing the gel at 10°C in a refrigerator until further use.

7. The method of claim 6, further comprising:
purging a chamber with nitrogen to remove oxygen while making the gelatin solution, the second solution and adding the THPC to form a normoxic N-(Hydroxymethyl)acrylamide (NHMA) polymer gel.

8. The method of claim 6 or 7, wherein 3 % by weight of gelatin, 0.02 mM of Hydroquinone, and 3 % by weight ofN,N-methylene-bis-acrylamide (BIS) are used.

9. The method of any one of claims 6 to 8, wherein 4-8 % by weight of N-(Hydroxymethyl)acrylamide (NHMA) is used.

10. The method of any one of claims 6 to 9, wherein 5 mM of
Tetrakis (hydroxymethyl)phosphonium chloride (THPC) is added.

11. 3D dosimeter comprising the polymerizable composition according to any of claims 1 to 5.

12. Use of the polymerizable composition according to any of claims 1 to 5 for measuring radiation doses.

13. The use of claim 12 for three-dimensionally measuring radiation dose distributions.

## Patentansprüche

1. Polymerisierbare Zusammensetzung zur Verwendung in einem 3D-Geldosimeter, umfassend:
2-5 Gew.-% Gelatine;
1-12 Gew.-% N-(Hydroxymethyl)acrylamid (NHMA);
1-4 Gew.-% N,N-Methylenbisacrylamid (BIS);
0-0,1 mM (mmol/l) Hydrochinon (HQ) und
hochreines entionisiertes Wasser als Lösungsmittel.

2. Zusammensetzung nach Anspruch 1, weiterhin umfassend:
1-50 mM Tetrakis(hydroxymethyl)phosphoniumchlorid (THPC).

3. Zusammensetzung nach Anspruch 1 oder 2, umfassend 3 Gew.-% Gelatine, 0,02 mM Hydrochinon und 3 Gew.-% N,N-Methylenbisacrylamid (BIS).

4. Zusammensetzung nach einem der vorangehenden Ansprüche, umfassend 4-8 Gew.-% N-(Hydroxymethyl)acrylamid (NHMA).

5. Zusammensetzung nach Anspruch 2, umfassend 5 mM Tetrakis(hydroxymethyl)-phosphoniumchlorid (THPC).

6. Verfahren zur Herstellung einer polymerisierbaren Zusammensetzung, umfassend:
Einweichen von 2-5 Gew.-% Gelatine in entionisiertem Wasser für 10 Minuten, um ein Gemisch aus der Gelatine und entionisiertem Wasser herzustellen;
Erhitzen des Gemischs aus der Gelatine und dem entionisierten Wasser für 1 Stunde bei 50 °C, um eine Gelatinelösung herzustellen;
Abkühlen der Gelatinelösung auf 40 °C;
Zugeben von 1-12 Gew.-% N-(Hydroxymethyl)acrylamid (NHMA) und 0-0,1 mM Hydrochinon (HQ) zu der Gelatinelösung und Mischen, um eine zweite Lösung herzustellen;
Abkühlen der zweiten Lösung auf 35°C; und
Zugeben von 1-50 mM Tetrakis(hydroxymethyl)phosphoniumchlorid (THPC) zu der zweiten Lösung und Ausbilden eines polymerisierbaren N-(hydroxymethyl)acrylamid-(NHMA)-Gels und Aufbewahren des Gels bei 10 °C in einem Kühlschrank bis zur weiteren Verwendung.

7. Verfahren nach Anspruch 6, weiterhin umfassend:
Spülen einer Kammer mit Stickstoff, um Sauerstoff zu entfernen, während des Herstellens der Gelatinelösung, der zweiten Lösung und des Zugebens des THPC, um ein normoxisches N-(Hydroxymethyl)acrylamid-(NHMA)-Polymergel auszubilden.

8. Verfahren nach Anspruch 6 oder 7, wobei 3 Gew.-% Gelatine, 0,02 mM Hydrochinon und 3 Gew.-% N,N-Methylenbisacrylamid (BIS) verwendet werden.

9. Verfahren nach einem der Ansprüche 6 bis 8, wobei 4-8 Gew.-% N-(Hydroxymethyl)-acrylamid (NHMA) verwendet werden.

10. Verfahren nach einem der Ansprüche 6 bis 9, wobei 5 mM Tetrakis(hydroxymethyl)-phosphoniumchlorid (THPC) zugegeben werden.

11. 3D-Dosimeter, das die polymerisierbare Zusammensetzung nach einem der Ansprüche 1 bis 5 umfasst.

12. Verwendung der polymerisierbaren Zusammensetzung nach einem der Ansprüche 1 bis 5 zum Messen von Strahlungsdosen.

13. Verwendung nach Anspruch 12 zum dreidimensionalen Messen von Strahlungsdosisverteilungen.

## Revendications

1. Une composition polymérisable pour l'utilisation dans un dosimètre pour gel 3D, comprenant :
2-5 % massique de gélatine ;
1-12 % massique de N-(hydroxyméthyl)acrylamide (NHMA) ;
1-4 % massique de N,N-méthylène-bis-acrylamide (BIS) ;
0-0,1 mM (mmol/l) d'hydroquinone (HQ) et
de l'eau désionisée ultra-pure, comme solvant.

2. La composition de la revendication 1, comprenant en outre :
1-50 mM de chlorure de tétrakis(hydroxyméthyl)phosphonium (THPC).

3. La composition selon la revendication 1 ou 2, comprenant 3 % massique de gélatine, 0,02 mM d'hydroquinone et 3 % massique de N,N-méthylène-bis-acrylamide (BIS).

4. La composition selon l'une des revendications précédentes, comprenant 4-8 % massique de N-(hydroxyméthyl)acrylamide (NHMA).

5. La composition de la revendication 2, comprenant 5 mM de chlorure de tétrakis(hydroxyméthyl)phosphonium (THPC).

6. Procédé de fabrication d'une composition polymérisable, comprenant :
tremper 2-5 % massique de gélatine pendant 10 menus dans de l'eau désionisée pour réaliser un mélange de gélatine et d'eau désionisée ;
chauffer le mélange de gélatine et d'eau désionisée pendant 1 heure à 50 °C pour réaliser une solution de gélatine ;
refroidir la solution de gélatine à 40 °C ;
ajouter à la solution de gélatine 1-12% massique de N-(hydroxyméthyl)acrylamide (NHMA) et 0-0,1 mM d'hydroquinone (HQ) et mélanger pour réaliser une deuxième solution ;
refroidir la deuxième solution à 35 °C ; et
ajouter à la deuxième solution 1-50 mM de chlorure de tétrakis(hydroxyméthyl)phosphonium (THPC) et former un gel polymérisable de N-(hydroxyméthyl)acrylamide (NHMA) et stocker le gel à 10 °C dans un réfrigérateur jusqu'à sa prochaine utilisation.

7. Le procédé de la revendication 6, comprenant en outre :
purger une chambre avec de l'azote pour éliminer l'oxygène lors de la réalisation de la solution de gélatine et de la deuxième solution, et ajouter le THPC pour former un gel polymère normoxique de N-(hydroxyméthyl)acrylamide (NHMA).

8. Le procédé de la revendication 6 ou 7, dans lequel 3 % massique de gélatine, 0,02 mM d'hydroquinone et 3 % massique de N,N-méthylène-bis-acrylamide (BIS) étant employés.

9. Le procédé selon l'une quelconque des revendications 6 à 8, dans lequel 4-8 % massique de N-(hydroxyméthyl)acrylamide (NHMA) étant employés.

10. Le procédé selon l'une quelconque des revendications 6 à 9, dans lequel 5 mM de chlorure de tétrakis(hydroxyméthyl)phosphonium (THPC) étant ajoutés.

11. Dosimètre 3D comprenant la composition polymérisable selon l'une quelconque des revendications 1 à 5.

12. Utilisation de la composition polymérisable selon l'une quelconque des revendications 1 à 5 pour mesurer des doses de rayonnement.

13. Utilisation de la revendication 12 pour effectuer des mesures tridimensionnelles de distributions de dose de rayonnement
